# EUROPEAN PATENT SPECIFICATION

(11) **EP 1 482 938 B1**
(45) Date of publication and mention of the grant of the patent: **08.08.2007**
(21) Application number: 03708130.4
(22) Date of filing: 25.02.2003
(51) Int. Cl.: A61K 31/4425, A61K 31/4523, A61K 31/4375, A61K 45/06, A61P 11/06, A61P 11/08

(54) **PHARMACEUTICAL COMPOSITION OF A PDE4 OR A PDE3/4 INHIBITOR AND A HISTAMINE RECEPTOR ANTAGONIST**
PHARMAZEUTISCHE ZUSAMMENSETZUNG ENTHALTEND EINEN PDE4 ODER PDE3/4 INHIBITOR UND EINE HISTAMINREZEPTORANTAGONISTEN
COMPOSITION PHARMACEUTIQUE CONTENANT UN INHIBITEUR DE LA PDE4 OU UN INHIBITEUR DE LA PDE3/4 ET UN ANTAGONISTE DES RECEPTEURS HISTAMINIQUES

(30) Priority: 06.03.2002 EP 02004987
(43) Date of publication of application: 08.12.2004
(73) Proprietor: Nycomed GmbH, 78467 Konstanz (DE)
(72) Inventor: BEUME, Rolf, 78465 Konstanz (DE); BUNDSCHUH, Daniela, CH - 8272 Ermatingen (CH); WEIMAR, Christian, 78467 Konstanz (DE); WOLLIN, Stefan-Lutz, 88709 Meersburg (DE)
(74) Representative: Wild, Robert
(86) International application number: PCT/EP2003/001876
(87) International publication number: WO 2003/074055

(56) References cited:
- EP-A- 1 005 865
- WO-A-01/30777
- WO-A-03/000289
- US-A- 5 962 464
- ANDRI L ET AL: "Combined treatment of allergic rhinitis with terfenadine and nimesulide, a non-steroidal antiinflammatory drug" ALLERGIE ET IMMUNOLOGIE, NOUVELLES EDITIONS MEDICALES FRANCAISES, PARIS, FR, vol. 24, no. 8, October 1992 (1992-10), pages 313-314,317-319, XP002102388 ISSN: 0397-9148
- HATZELMANN A ET AL: "ANTI-INFLAMMATORY AND IMMUNOMODULATORY POTENTIAL OF THE NOVEL PDE4 INHIBITOR ROFLUMILAST IN VITRO" JOURNAL OF PHARMACOLOGY AND EXPERIMENTAL THERAPEUTICS, AMERICAN SOCIETY FOR PHARMACOLOGY AND, US, vol. 297, no. 1, 2001, pages 267-279, XP001024814 ISSN: 0022-3565

## Description

### Field of application of the invention

The present invention relates to combinations of pharmaceutically active substances for use in the treatment of respiratory diseases.

The substances used in the combinations according to the invention are known active compounds from the PDE4 and PDE3/4 inhibitors class and active compounds from the class of the histamine receptor antagonists.

### Known technical background

In the international application WO03/000289 compositions are described which comprise a PDE4 inhibitor and a H1-receptor antagonist and the use of these compositions for the manufacture of a medicament for the treatment of respiratory diseases. In the European patent application EP1005865 pharmaceutical compositions comprising Nimesulide and Cetirizine are disclosed. In Allergie et Immunologie, Vol 24, No 8, 1992, pp 313-319, Andri L. et al have described a combination of Nimesulide with Terfenadine for the treatment of allergic rhinitis. In J. Pharmacol. Exp. Therapeut. Vol 297, No 1, 2001, pp 267-279, Hatzelmann A. et al. have described the anti-inflammatory and immunomodulatory potential of the novel PDE4 inhibitor Roflumilast in vitro.

### Description of the invention

Asthma is a common inflammatory disease of the respiratory tract, accounting for 1 - 3% of all office visits, 500,000 hospital admissions per year and more pediatric hospital admissions than any other single illness in the US. Annually, more than 5000 children and adults die of asthma attacks in the United States (William E. S.; Goodmann Gilmann A.:The pharmacological Basis of Therapeutics, 9^{th} Edition, pp. 152 & 659-682, Mc Graw Hill, New York 1996).

Asthma can no longer be viewed simply as a reversible airway obstruction. It should instead be considered primarily as an inflammatory illness that has bronchial hyperactivity and bronchospasm as its results. Allergen specific immunoglobulin E (IgE) is bound to the mast cells via Fc receptors. It is a fragment obtained by papain digestion of immunoglobulin molecules and contains most of the antigenic determinants. When an allergen comes into contact with IgE, the mast cells are activated and release a number of inflammatory mediators, which include granule contents like histamine, proteases, heparin, and tumor necrosis factor (TNF), a variety of lipid membrane derived molecules like prostaglandins, leukotrienes and platelet activating factor (PAF), and a number of cytokines like interleukin (IL)-1, 3, 4, 5, 6 and 8 and chemokines. An enormous variety of mediators are released which have more than one potent effect on airway inflammation.

As a result of vasodilation, increased vasopermeability and increased endothelial adhesiveness towards leukocytes further leads to an influx of inflammatory cells like lymphocytes, eosinophils and macrophages from blood circulation into the tissues. This in turn leads to the release of mediators which have further inflammatory effects (Rao A. R. et al. Recent Perspectives in the design of antiasthmatic agents, Pharmazie, 55, 7, 475-482, 2000).

Thus, it can be understood that it is unlikely that drugs affecting a single mediator can satisfactorily treat the disease alone. As asthma is one of the major diseases affecting mankind, there is a need to develop drugs which can affect a wide variety of mediators.

Therefore, it is the object of the present invention to make available respiratory tract therapeutics which fulfill the following conditions:
- Favorable simultaneous influence on several of the inflammatory mediators
- Marked bronchorelaxation and -dilatation
- Good oral availability
- Minor side effects
- Good suitability for long-term therapy
- Favorable influence on bronchial hyperreactivity

It has now been found that the combined use of a PDE4 or a PDE3/4 inhibitor and a histamine receptor antagonist outstandingly fulfills the above-mentioned conditions.

The invention thus relates to the combined use of a PDE4 or a PDE3/4 inhibitor and a histamine receptor antagonist in the treatment of respiratory diseases.

By the expression "PDE4 inhibitor" is meant a selective phosphodiesterase inhibitor, which inhibits preferentially the type 4 phosphodiesterase when compared to other known types of phosphodiesterase, e.g. type 1, 2, 3, 5 etc., whereby the compound has a lower IC₅₀ (more potent) for the type 4 phosphodiesterase, such as where the IC₅₀ for PDE4 inhibition is about factor 10 lower compared to IC₅₀ for inhibition of other known type of phosphodiesterase, e.g. type 1, 2, 3, 5 etc.

Analogously, the expression "PDE3/4 inhibitor" is defined. Methods to determine the activity and selectivity of a phosphodiesterase inhibitor are known to the person skilled in the art. In this connection it may be mentioned, for example, the methods described by Thompson et al. (Adv Cycl Nucl Res 10: 69-92, 1979), Giembycz et al. (Br J Pharmacol 118: 1945-1958, 1996) and the phosphodiesterase scintillation proximity assay of Amersham Pharmacia Biotech.

By the expression "histamine receptor antagonist" are meant H₁-receptor antagonists, particularly the so called H₁-receptor antagonists of the second generation (Mutschler, Arzneimittelwirkungen, 8. Edition, 2001, pages 456-461).

Exemplary PDE inhibitors are shown with the aid of their formulae:

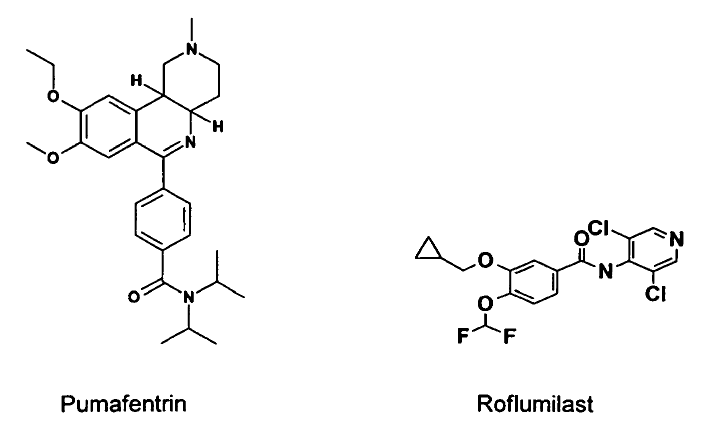

In the above formulae there is given neither any stereochemical information nor are hydrogen atoms indicated [-O is accordingly -OH, >N is >NH or -N is NH₂]. Methyl groups, e.g. on the oxygen atoms, are indicated by lines].

Substances having good oral availability are preferred here.

Preferred PDE4 or PDE3/4 inhibitors are 3-Cyclopropylmethoxy-4-difluoromethoxy-N-(3,5-dichloropyrid-4-yl)-benzamide [INN: ROFLUMILAST], (-)-cis-9-ethoxy-8-methoxy-2-methyl-1,2,3,4,4a,10b-hexahydro-6-(4-diisopropylaminocarbonylphenyl)-benzo-[c][1,6]naphthyridine [INN: PUMAFENTRINE] and their pharmaceutically acceptable derivatives.

The preferred histamine receptor antagonist is (plus/minus)-[2-[4-(p-chloro-alpha-phenylbenzyl)-1-piperazinyl]ethoxy]-acetic acid [INN: CETIRIZINE] and its pharmaceutically acceptable derivatives.

In the context of the present invention, unless otherwise stated, a pharmaceutically acceptable derivative of an active ingredient means a pharmaceutically acceptable salt or solvate (e. g. hydrate), a pharmaceutically acceptable solvate of such salt, a pharmaceutically acceptable N-oxide or a pharmaceutically acceptable salt or solvate of the latter.

Suitable pharmacologically tolerable salts here are on the one hand in particular water-soluble and water-insoluble acid addition salts with acids such as, for example, hydrochloric acid, hydrobromic acid, phosphoric acid, nitric acid, sulfuric acid, acetic acid, citric acid, D-gluconic acid, benzoic acid, 2-(4-hydroxybenzoyl)-benzoic acid, butyric acid, sulfosalicylic acid, maleic acid, lauric acid, malic acid, fumaric acid, succinic acid, oxalic acid, tartaric acid, embonic acid, stearic acid, toluenesulfonic acid, methanesulfonic acid or 1-hydroxy-2-naphthoic acid, the acids being employed in salt preparation - depending on whether it is a mono- or polybasic acid and depending on which salt is desired- in an equimolar quantitative ratio or one differing therefrom. Furthermore, the active compounds mentioned can also be present as pure enantiomers or as enantiomer mixtures in any mixing ratio.

On the other hand, salts with bases are also suitable. Examples of salts with bases which may be mentioned are alkali metal (lithium, sodium, potassium) or calcium, aluminium, magnesium, titanium, ammonium, meglumine or guanidinium salts, where here too the bases are employed in salt preparation in an equimolar quantitative ratio or one differing therefrom.

Certain of the active ingredients used in the present invention are capable of existing in stereoisomeric forms. The invention encompasses all stereoisomers of the active ingredients and mixtures thereof including racemates. Tautomers and mixtures thereof of the active ingredients are also part of the present invention.

In accordance with the present invention, there is provided in a first aspect a pharmaceutical composition comprising, in admixture, a first active ingredient which is selected from the group consisting of 3-Cyclopropylmethoxy-4-difluoromethoxy-N-(3,5-dichloropyrid-4-yl)-benzamide [INN: ROFLUMILAST], (-)-cis-9-ethoxy-8-methoxy-2-methyl-1,2,3,4,4a,10b-hexahydro-6-(4-diisopropylaminocarbonylphenyl)-benzo-[c][1,6]naphthyridine [INN: PUMAFENTRINE] and their pharmaceutically acceptable derivatives, and a second active ingredient, which is selected from the group consisting of (plus/minus)-[2-[4-(p-chloro-alpha-phenylbenzyl)-1-piperazinyl]ethoxy]-acetic acid [INN: CETIRIZINE] and its pharmaceutically acceptable derivatives.

In a second aspect - which is an embodiment of the first aspect - there is provided a pharmaceutical composition, wherein the first active ingredient is selected from the group consisting of 3-Cyclopropylmethoxy-4-difluoromethoxy-N-(3,5-dichloropyrid-4-yl)-benzamide [INN: ROFLUMILAST], a pharmaceutically acceptable salt, solvate or N-oxide thereof, or solvate of an salt or N-oxide thereof.

In a third aspect - which is another embodiment of the first aspect - there is provided a pharmaceutical composition, wherein the first active ingredient is selected from the group consisting of 3-Cyclopropylmethoxy-4-difluoromethoxy-N-(3,5-dichloropyrid-4-yl)-benzamide [INN: ROFLUMILAST] or a pharmaceutically acceptable salt, solvate or N-oxide thereof, or solvate of an salt or N-oxide thereof, and the second active ingredient is selected from the group consisting of (plus/minus)-[2-[4-(p-chloro-alphaphenylbenzyl)-1-piperazinyl]ethoxy]-acetic acid [INN: CETIRIZINE] and its pharmaceutically acceptable derivatives.

In a fourth aspect the invention provides a pharmaceutical product comprising, in combination, a preparation of a first active ingredient which is selected from the group consisting of 3-Cyclopropylmethoxy-4-difluoromethoxy-N-(3,5-dichloropyrid-4-yl)-benzamide [INN: ROFLUMILAST], (-)-cis-9-ethoxy-8-methoxy-2-methyl-1,2,3,4,4a,10b-hexahydro-6-(4-diisopropylaminocarbonylphenyl)-benzo-[c][1,6]-naphthyridine [INN: PUMAFENTRINE] and their pharmaceutically acceptable derivatives, and a preparation of a second active ingredient which is selected from the group consisting of (plus/minus)-[2-[4-(p-chloro-alpha-phenylbenzyl)-1-piperazinyl]ethoxy]-acetic acid [INN: CETIRIZINE] and its pharmaceutically acceptable derivatives for simultaneous, sequential or separate use in therapy.

In a fifth aspect - which is an embodiment of the fourth aspect - the invention provides a pharmaceutical product, wherein the active ingredient in the preparation of the first active ingredient is selected from the group consisting of 3-Cyclopropylmethoxy-4-difluoromethoxy-N-(3,5-dichloropyrid-4-yl)-benzamide [INN: ROFLUMILAST] or a pharmaceutically acceptable salt, solvate or N-oxide thereof, or solvate of a salt of N-oxide thereof.

In the sixth aspect, which is a still further embodiment of the fourth aspect - the invention provides a pharmaceutical product wherein the first active ingredient is selected from the group consisting of 3-Cyclopropylmethoxy-4-difluoromethoxy-N-(3,5-dichloropyrid-4-yl)-benzamide [INN: ROFLUMILAST] or a pharmaceutically acceptable salt, solvate or N-oxide thereof, or solvate of a salt of N-oxide thereof and the second active ingredient is selected from the group consisting of (plus/minus)-[2-[4-(p-chloro-alpha-phenylbenzyl)-1-piperazinyl]ethoxy]-acetic acid [INN: CETIRIZINE] and its pharmaceutically acceptable derivatives.

In a seventh aspect the invention provides a kit comprising a preparation of a first active ingredient which is selected from the group consisting of 3-Cyclopropylmethoxy-4-difluoromethoxy-N-(3,5-dichloropyrid-4-yl)-benzamide [INN: ROFLUMILAST], (-)-cis-9-ethoxy-8-methoxy-2-methyl-1,2,3,4,4a,10b-hexahydro-6-(4-diisopropylaminocarbonylphenyl)-benzo-[c][1,6]naphthyridine [INN: PUMAFENTRINE] and their pharmaceutically acceptable derivatives, a preparation of a second active ingredient which is selected from the group consisting of (plus/minus)-[2-[4-(p-chloro-alpha-phenylbenzyl)-1-piperazinyl]-ethoxy]-acetic acid [INN: CETIRIZINE] and its pharmaceutically acceptable derivatives, and instructtions for the simultaneous, sequential or separate administration of the preparations to a patient in need thereof.

In a eighth aspect - which is an embodiment of the seventh aspect - the invention provides a kit wherein the active ingredient in the preparation of the first active ingredient is selected from the group consisting of 3-Cyclopropylmethoxy-4-difluoromethoxy-N-(3,5-dichloropyrid-4-yl)-benzamide [INN: ROFLUMILAST], a pharmaceutically acceptable salt, solvate or N-oxide thereof, or solvate of an salt or N-oxide thereof.

In a ninth aspect - which is still another embodiment of the seventh aspect - the invention provides a kit wherein the first active ingredient is selected from the group consisting of 3-Cyclopropylmethoxy-4-difluoromethoxy-N-(3,5-dichloropyrid-4-yl)-benzamide [INN: ROFLUMILAST] or a pharmaceutically acceptable salt, solvate or N-oxide thereof, or solvate of an salt or N-oxide thereof, and the second active ingredient is selected from the group consisting of (plus/minus)-[2-[4-(p-chloro-alpha-phenylbenzyl)-1-piperazinyl]ethoxy]-acetic acid [INN: CETIRIZINE] and its pharmaceutically acceptable derivatives.

It has been found that the administration of active ingredients according to the invention is advantageous because it results - in comparison to the administration of a single active ingredient from the PDE4 and PDE3/4 inhibitors or the histamine receptor antagonists class - in a reduced early allergic response and/or in a reduced late inflammatory airway response.

The pharmaceutical composition of the present invention may be prepared by mixing the first active ingredient with the second active ingredient. Therefore, in the tenth aspect of the present invention, there is provided a process for the preparation of a pharmaceutical composition which comprises mixing a first active ingredient which is selected from the group consisting of 3-Cyclopropylmethoxy-4-difluoromethoxy-N-(3,5-dichloropyrid-4-yl)-benzamide [INN: ROFLUMILAST], (-)-cis-9-ethoxy-8-methoxy-2-methyl-1,2,3,4,4a,10b-hexahydro-6-(4-diisopropylaminocarbonylphenyl)-benzo-[c][1,6]naphthyridine [INN: PUMAFENTRINE] and their pharmaceutically acceptable derivatives with a second active ingredient which is selected from the group consisting of (plus/minus)-[2-[4-(p-chloro-alpha-phenylbenzyl)-1-piperazinyl]ethoxy]-acetic acid [INN: CETIRIZINE] and its pharmaceutically acceptable derivatives.

In an eleventh aspect - which is an embodiment of the tenth aspect - there is provided a process for the preparation of a pharmaceutical composition which comprises mixing a first active ingredient which is selected from the group consisting of 3-Cyclopropylmethoxy-4-difluoromethoxy-N-(3,5-dichloropyrid-4-yl)-benzamide [INN: ROFLUMILAST] or a pharmaceutically acceptable salt, solvate or N-oxide thereof, or solvate of a salt or N-oxide thereof, with a second active ingredient which is selected from the group consisting of (plus/minus)-[2-[4-(p-chloro-alpha-phenylbenzyl)-1-piperazinyl]ethoxy]-acetic acid [INN: CETIRIZINE] and its pharmaceutically acceptable derivatives.

The first and second active ingredient may alternatively (other than in admixture as described above) be administered simultaneously, sequentially or separately to treat respiratory diseases. By sequential is meant that the first and second active ingredient are administered one immediately after the other. Separately means a time difference of administration of up to 24 h, preferably up to 12 h.

The present invention further provides the use of a pharmaceutical composition, or pharmaceutical product according to the invention in the manufacture of a medicament for the prophylaxis and/or treatment of a respiratory disease.

Respiratory diseases which may be mentioned are in particular allergen- and inflammation- induced bronchial disorders (bronchitis, obstructive bronchitis, spastic bronchitis, allergic bronchitis, allergic asthma, bronchial asthma, COPD, allergic, seasonal and perennial rhinitis), which can be treated by the combination according to the invention also in the sense of a long-term therapy (if desired with appropriate adjustment of the dosage of the individual components to the needs at the time, for example needs subject to seasonally related variations).

The active ingredients may, and indeed will, as part of the pharmaceutical composition, the Pharmaceutical product or preparation, be used in admixture with one or more pharmaceutically acceptable auxiliaries and/or excipients.

Within the meaning of the present invention, "use" is preferably understood as meaning the oral administration of both active ingredients. The active ingredients also can be administered as a nasal spray, an aerosol, or as an inhaled powder. Further methods of administration, which may be mentioned are the parenteral (e. g. intravenous) and the transdermal administration of the active ingredients.

The invention encompasses on the one hand co-administering both drugs in one delivery form such as a fixed oral combination (putting both active ingredients in one tablet), as an inhaler (putting both active ingredients in the same inhaler) or as a free oral combination (putting both active ingredients in two separate tablets). On the other hand it encompasses also the administration of the drugs in two different delivery forms such as putting the PDE4 inhibitor into tablets and package them with an inhaler that contains the histamine receptor antagonist, or vice versa.

The person skilled in the art is familiar on the basis of his/her expert knowledge with which excipients or auxiliaries are suitable for the desired pharmaceutical composition, product or preparation. In addition to solvents, gel-forming agents, tablet excipients and other active compound carriers, it is possible to use, for example, antioxidants, dispersants, emulsifiers, antifoams, flavor corrigents, preservatives, solubilizers, colorants or permeation promoters and complexing agents (e.g. cyclodextrins).

The pharmaceutical compositions or preparations according to the invention for oral administration are preferably in the form of tablets, coated tablets, capsules, emulsions, suspensions or solutions, the active ingredient content advantageously being between 0.1 and 95% and by appropriate choice of the excipients and the auxiliaries, it being possible to achieve a pharmaceutical administration form precisely tailored to the active ingredient(s) and/or to the desired onset of action (e.g. a sustained release form or an enteric form).

The active ingredients according to the invention are administered by inhalation preferably in the form of an aerosol; the aerosol particles of solid, liquid or mixed composition preferably having a diameter of 0.5 to 10 µm, advantageously of 2 to 6 µm.

Aerosol generation can be carried out, for example, by pressure-driven jet atomizers or ultrasonic atomizers, but advantageously by propellant-driven metered aerosols or propellant-free administration of micronized active compounds from inhalation capsules.

Depending on the inhaler system used, in addition to the active compounds the administration forms additionally contain the required excipients, such as, for example, propellants (e.g. Frigen in the case of metered aerosols), surface-active substances, emulsifiers, stabilizers, preservatives, flavorings, fillers (e.g. lactose in the case of powder inhalers) or, if appropriate, further active compounds.

For the purposes of inhalation, a large number of apparatuses are available with which aerosols of optimum particle size can be generated and administered, using an inhalation technique which is as right as possible for the patient. In addition to the use of adaptors (spacers, expanders) and pear-shaped containers (e.g. Nebulator®, Volumatic®), and automatic devices emitting a puffer spray (Autohaler®), for metered aerosols, in particular in the case of powder inhalers, a number of technical solutions are available (e.g. Diskhaler®, Rotadisk®, Turbohaler® or the inhaler described in European Patent Application EP 0 505 321), using which an optimal administration of active compound can be achieved.

Typical compositions for nasal delivery include those mentioned above for inhalation and further include non-pressurized compositions in the form of a solution or suspension in an inert vehicle such as water optionally in combination with conventional excipients such as buffers, anti-microbials, tonicity modifying agents and viscosity modifying agents which may be administered by nasal pump.

Typical transdermal formulations comprise a conventional aqueous or non aqueous vehicle, for example a cream, ointment, lotion or paste, or are in the form of a medicated plaster, patch or membrane.

For the above-mentioned therapeutic uses the dosages administered will, of course, vary with the first and second active ingredients employed, the mode of administration, the treatment desired and the disorder indicated.

However, in general, satisfactory results will be obtained when the total daily dosage of first active ingredient(s), the PDE4 respectively the PDE3/4 inhibitors, when taken oral is in the range from 1 - 2000 µg/kg of body weight. In the case of the particularly preferred PDE4 inhibitor ROFLUMILAST, the daily dosage is in a range from 1-20 µg/kg of body weight. The daily dosage for the particularly preferred PDE3/4 inhibitor PUMAFENTRINE is in a range from 300- 1500 µg/kg of body weight.

The total daily dosage of the second active ingredient(s), the histamine receptor antagonists also can vary within a wide range (0.1- 1500 µg/kg of body weight).

## Claims

1. A pharmaceutical composition comprising, in admixture, a first active ingredient which is selected from the group consisting of 3-Cyclopropylmethoxy-4-difluoromethoxy-N-(3,5-dichloropyrid-4-yl)-benzamide [INN: ROFLUMILAST], (-)-cis-9-ethoxy-8-methoxy-2-methyl-1,2,3,4,4a,10b-hexahydro-6-(4-diisopropylaminocarbonylphenyl)-benzo-[c][1,6]naphthyridine [INN: PUMAFENTRINE] and their pharmaceutically acceptable derivatives, and a second active ingredient, which is selected from the group consisting of (plus/minus)-[2-[4-(p-chloro-alpha-phenylbenzyl)-1-piperazinyl]ethoxy]-acetic acid [INN: CETIRIZINE] and its pharmaceutically acceptable derivatives.

2. A pharmaceutical composition according to claim 1, wherein the first and/or second active ingredient is in the form of a pharmaceutically acceptable salt, solvate, N-oxide, or solvate of a salt or N-oxide.

3. A pharmaceutical composition according to claim 1, wherein the first active ingredient is selected from the group consisting of 3-Cyclopropylmethoxy-4-difluoromethoxy-N-(3,5-dichloropyrid-4-yl)-benzamide [INN: ROFLUMILAST], a pharmaceutically acceptable salt, solvate or N-oxide thereof, or solvate of an salt or N-oxide thereof.

4. A pharmaceutical composition according to claim 1, wherein the first active ingredient is selected from the group consisting of 3-Cyclopropylmethoxy-4-difluoromethoxy-N-(3,5-dichloropyrid-4-yl)-benzamide [INN: ROFLUMILAST] or a pharmaceutically acceptable salt, solvate or N-oxide thereof, or solvate of an salt or N-oxide thereof, and the second active ingredient is selected from the group consisting of (plus/minus)-[2-[4-(p-chloro-alpha-phenylbenzyl)-1-piperazinyl]ethoxy]-acetic acid [INN: CETIRIZINE] and its pharmaceutically acceptable derivatives.

5. A pharmaceutical composition according to any of claims 1 to 4, which is a fixed oral combination.

6. Use of a pharmaceutical composition according to any of claims 1 to 5 in the manufacture of a medicament for the treatment of respiratory diseases.

7. A process for the preparation of a pharmaceutical composition as defined in any of claims 1 to 5, which comprises mixing the first active ingredient with the second active ingredient.

8. A pharmaceutical product comprising in combination, a preparation of a first active ingredient which is selected from the group consisting of 3-Cyclopropylmethoxy-4-difluoromethoxy-N-(3,5-dichloropyrid-4-yl)-benzamide [INN: ROFLUMILAST], (-)-cis-9-ethoxy-8-methoxy-2-methyl-1,2,3,4,4a,10b-hexahydro-6-(4-diisopropylaminocarbonylphenyl)-benzo-[c][1,6]naphthyridine [INN: PUMAFENTRINE] and their pharmaceutically acceptable derivatives, and a preparation of a second active ingredient, which is selected from the group consisting of (plus/minus)-[2-[4-(p-chloro-alpha-phenylbenzyl)-1-piperazinyl]ethoxy]-acetic acid [INN: CETIRIZINE] and its pharmaceutically acceptable derivatives for simultaneous, sequential or separate use in therapy.

9. A pharmaceutical product according to claim 8, wherein the first and/or second active ingredient is in the form of a pharmaceutically acceptable salt, solvate, N-oxide, or solvate of a salt or N-oxide.

10. A pharmaceutical product according to claim 8, wherein the first active ingredient is selected from the group consisting of 3-Cyclopropylmethoxy-4-difluoromethoxy-N-(3,5-dichloropyrid-4-yl)-benzamide [INN: ROFLUMILAST], a pharmaceutically acceptable salt, solvate or N-oxide thereof, or solvate of an salt or N-oxide thereof.

11. A pharmaceutical product according to claim 8, wherein the first active ingredient is selected from the group consisting of 3-Cyclopropylmethoxy-4-difluoromethoxy-N-(3,5-dichloropyrid-4-yl)-benzamide [INN: ROFLUMILAST] or a pharmaceutically acceptable salt, solvate or N-oxide thereof, or solvate of an salt or N-oxide thereof, and the second active ingredient is selected from the group consisting of (plus/minus)-[2-[4-(p-chloro-alpha-phenylbenzyl)-1-piperazinyl]ethoxy]-acetic acid [INN: CETIRIZINE] and its pharmaceutically acceptable derivatives.

12. Use of a pharmaceutical product according to any of claims 8 to 11 in the manufacture of a medicament for the treatment of respiratory diseases.

13. A kit comprising a preparation of a first active ingredient, which is selected from the group consisting of 3-Cyclopropylmethoxy-4-difluoromethoxy-N-(3,5-dichloropyrid-4-yl)-benzamide [INN: ROFLUMILAST], (-)-cis-9-ethoxy-8-methoxy-2-methyl-1,2,3,4,4a,10b-hexahydro-6-(4-diisopropylaminocarbonylphenyl)-benzo-[c][1,6]naphthyridine [INN: PUMAFENTRINE] and their pharmaceutically acceptable derivatives, a preparation of a second active ingredient which is selected from the group consisting of (plus/minus)-[2-[4-(p-chloro-alpha-phenylbenzyl)-1-piperazinyl]ethoxy]-acetic acid [INN: CETIRIZINE] and its pharmaceutically acceptable derivatives, and instructions for the simultaneous, sequential or separate administration of the preparations to a patient in need thereof.

14. A kit according to claim 13, wherein the first and/or second active ingredient is in the form of a pharmaceutically acceptable salt, solvate, N-oxide, or solvate of a salt or N-oxide.

15. A kit according to claim 13, wherein the first active ingredient is selected from the group consisting of 3-Cyclopropylmethoxy-4-difluoromethoxy-N-(3,5-dichloropyrid-4-yl)-benzamide [INN: ROFLUMILAST], a pharmaceutically acceptable salt, solvate or N-oxide thereof, or solvate of an salt or N-oxide thereof.

16. A kit according to claim 13, wherein the first active ingredient is selected from the group consisting of 3-Cyclopropylmethoxy-4-difluoromethoxy-N-(3,5-dichloropyrid-4-yl)-benzamide [INN: ROFLUMILAST] or a pharmaceutically acceptable salt, solvate or N-oxide thereof, or solvate of an salt or N-oxide thereof, and the second active ingredient is selected from the group consisting of (plus/minus)-[2-[4-(p-chloro-alpha-phenylbenzyl)-1-piperazinyl]ethoxy]-acetic acid [INN: CETIRIZINE] and its pharmaceutically acceptable derivatives.

17. Use according to any of claims 6 or 12, wherein the respiratory disease is selected from bronchitis, obstructive bronchitis, spastic bronchitis, allergic bronchitis, allergic asthma, bronchial asthma, COPD, and allergic, seasonal or perennial rhinitis.

## Patentansprüche

1. Pharmazeutische Zusammensetzung enthaltend, als Mischung, einen ersten Wirkstoff, ausgewählt aus der aus 3-Cyclopropylmethoxy-4-difluormethoxy-N-(3,5-dichlorpyrid-4-yl)benzamid [INN: ROFLUMILAST], (-)-cis-9-Ethoxy-8-methoxy-2-methyl-1,2,3,4,4a,10b-hexahydro-6-(4-diisopropylaminocarbonylphenyl)benzo-[c][1,6]naphthyridin [INN: PUMAFENTRINE] und deren pharmazeutisch unbedenklichen Derivaten bestehenden Gruppe und einem zweiten Wirkstoff ausgewählt aus der aus (+/-)-[2-[4-(p-Chlor-α-phenylbenzyl)-1-piperazinyl]ethoxy]essigsäure [INN: CETIRIZINE] und seinen pharmazeutisch unbedenklichen Derivaten bestehenden Gruppe.

2. Pharmazeutische Zusammensetzung nach Anspruch 1, wobei der erste und/oder der zweite Wirkstoff in Form eines pharmazeutisch unbedenklichen Salzes, Solvats, N-Oxids oder Solvats eines Salzes oder N-Oxids vorliegt.

3. Pharmazeutische Zusammensetzung nach Anspruch 1, wobei der erste Wirkstoff ausgewählt ist aus der aus 3-Cyclopropylmethoxy-4-difluormethoxy-N-(3,5-dichlorpyrid-4-yl)benzamid [INN: ROFLUMILAST], pharmazeutisch unbedenklichen Salzen, Solvaten oder N-Oxiden davon und Solvaten eines Salzes oder N-Oxids davon bestehenden Gruppe.

4. Pharmazeutische Zusammensetzung nach Anspruch 1, wobei der erste Wirkstoff ausgewählt ist aus der aus 3-Cyclopropylmethoxy-4-difluormethoxy-N-(3,5-dichlorpyrid-4-yl)benzamid [INN: ROFLUMILAST] und pharmazeutisch unbedenklichen Salzen, Solvaten oder N-Oxiden davon oder Solvaten eines Salzes oder N-Oxids davon bestehenden Gruppe und der zweite Wirkstoff ausgewählt ist aus der aus (+/-)-[2-[4-(p-Chlor-α-phenylbenzy)-1-piperazinyl]ethoxy]-essigsäure [INN: CETIRIZINE] und seinen pharmazeutisch unbedenklichen Derivaten bestehenden Gruppe.

5. Pharmazeutische Zusammensetzung nach einem der Ansprüche 1 bis 4, bei der es sich um eine fixe orale Kombination handelt.

6. Verwendung einer pharmazeutischen Zusammensetzung nach einem der Ansprüche 1 bis 5 bei der Herstellung eines Medikaments zur Behandlung von Atemwegserkrankungen.

7. Verfahren zur Herstellung einer pharmazeutischen Zusammensetzung nach einem der Ansprüche 1 bis 5, bei dem man den ersten Wirkstoff mit dem zweiten Wirkstoff mischt.

8. Pharmazeutisches Produkt enthaltend, als Kombination, eine Zubereitung eines ersten Wirkstoffs, ausgewählt aus der aus 3-Cyclopropylmethoxy-4-difluormethoxy-N-(3,5-dichlorpyrid-4-yl)benzamid [INN: ROFLUMILAST], (-)-cis-9-Ethoxy-8-methoxy-2-methyl-1,2,3,4,4a,10b-hexahydro-6-(4-diisopropylaminocarbonylphenyl)benzo-[c][1,6]naphthyridin [INN: PU-MAFENTRINE] und deren pharmazeutisch unbedenklichen Derivaten bestehenden Gruppe, und eine Zubereitung eines zweiten Wirkstoffs, ausgewählt aus der aus (+/-)-[2-[4-(p-Chlor-α-phenylbenzyl)-1-piperazinyl]ethoxy]essigsäure [INN: CETIRIZINE] und seinen pharmazeutisch unbedenklichen Derivaten bestehenden Gruppe, zur gleichzeitigen, aufeinanderfolgenden oder getrennten Verwendung in der Therapie.

9. Pharmazeutisches Produkt nach Anspruch 8, wobei der erste und/oder zweite Wirkstoff in Form eines pharmazeutisch unbedenklichen Salzes, Solvats, N-Oxids oder Solvats eines Salzes oder N-Oxids vorliegt.

10. Pharmazeutisches Produkt nach Anspruch 8, wobei der erste Wirkstoff ausgewählt ist aus der aus 3-Cyclopropylmethoxy-4-difluormethoxy-N-(3,5-dichlorpyrid-4-yl)benzamid [INN: ROFLUMILAST], pharmazeutisch unbedenklichen Salzen, Solvaten oder N-Oxiden davon und Solvaten eines Salzes oder N-Oxids davon bestehenden Gruppe.

11. Pharmazeutisches Produkt nach Anspruch 8, wobei der erste Wirkstoff ausgewählt ist aus der aus 3-Cyclopropylmethoxy-4-difluormethoxy-N-(3,5-dichlorpyrid-4-yl)benzamid [INN: ROFLUMILAST] und pharmazeutisch unbedenklichen Salzen, Solvaten oder N-Oxiden davon oder Solvaten eines Salzes oder N-Oxids davon bestehenden Gruppe und der zweite Wirkstoff ausgewählt ist aus der aus (+/-)-[2-[4-(p-Chlor-α-phenylbenzyl)-1-piperazinyl]ethoxy]essigsäure [INN: CETIRIZINE] und seinen pharmazeutisch unbedenklichen Derivaten bestehenden Gruppe.

12. Verwendung eines pharmazeutischen Produkts nach einem der Ansprüche 8 bis 11 bei der Herstellung eines Medikaments zur Behandlung von Atemwegserkrankungen.

13. Kit, enthaltend eine Zubereitung eines ersten Wirkstoffs, ausgewählt aus der aus 3-Cyclopropylmethoxy-4-difluormethoxy-N-(3,5-dichlorpyrid-4-yl)benzamid [INN: ROFLUMILAST], (-)-cis-9-Ethoxy-8-methoxy-2-methyl-1,2,3,4,4a, 10b-hexahydro-6-(4-diisopropylaminocarbonylphenyl)benzo-[c][1 ,6]naphthyridin [INN: PUMAFENTRINE] und deren pharmazeutisch unbedenklichen Derivaten bestehenden Gruppe, eine Zubereitung eines zweiten Wirkstoffs ausgewählt aus der aus (+/-)-[2-[4-(p-Chlor-α-phenylbenzyl)-1-piperazinyl]-ethoxy]essigsäure [INN: CETIRIZINE] und seinen pharmazeutisch unbedenklichen Derivaten bestehenden Gruppe, und Anweisungen für die gleichzeitige, aufeinanderfolgende oder getrennte Verabreichung der Zubereitungen an einen deren bedürftigen Patienten.

14. Kit nach Anspruch 13, wobei der erste und/oder zweite Wirkstoff in Form eines pharmazeutisch unbedenklichen Salzes, Solvats, N-Oxids oder Solvats eines Salzes oder N-Oxids vorliegt.

15. Kit nach Anspruch 13, wobei der erste Wirkstoff ausgewählt ist aus der aus 3-Cyclopropylmethoxy-4-difluormethoxy-N-(3,5-dichlorpyrid-4-yl)benzamid [INN: ROFLUMILAST], pharmazeutisch unbedenklichen Salzen, Solvaten oder N-Oxiden davon und Solvaten eines Salzes oder N-Oxids davon bestehenden Gruppe.

16. Kit nach Anspruch 13, wobei der erste Wirkstoff ausgewählt ist aus der aus 3-Cyclopropylmethoxy-4-difluormethoxy-N-(3,5-dichlorpyrid-4-yl)benzamid [INN: ROFLUMILAST], pharmazeutisch unbedenklichen Salzen, Solvaten oder N-Oxiden davon und Solvaten eines Salzes oder N-Oxids davon bestehenden Gruppe, und der zweite Wirkstoff ausgewählt ist aus der aus (+/-)-[2-[4-(p-Chlor-α-phenylbenzyl)-1-piperazinyl]ethoxy]essigsäure [INN: CETIRIZINE] und ihren pharmazeutisch unbedenklichen Derivaten bestehenden Gruppe.

17. Verwendung nach einem Ansprüche 6 oder 12, wobei die Atemwegserkrankung ausgewählt ist aus Bronchitis, obstruktiver Bronchitis, spastischer Bronchitis, allergischer Bronchitis, allergischem Asthma, Bronchialasthma, chronisch-obstruktiver Atemwegserkrankung (COPD), allergischer Rhinitis, saisonaler Rhinitis oder perennialer Rhinitis.

## Revendications

1. Composition pharmaceutique comprenant, en mélange, un premier principe actif qui est choisi dans le groupe constitué par le 3-cyclopropylméthoxy-4-difluorométhoxy-N-(3,5-dichloropyrid-4-yl)benzamide [DCI : ROFLUMILAST], la (-)-cis-9-éthoxy-8-méthoxy-2-méthyl-1,2,3,4,4a, 10b-hexahydro-6-(4-diisopropylaminocarbonylphényl)benzo-[c][1,6] naphtyridine [DCI : PUMAFENTRINE] et leurs dérivés pharmaceutiquement acceptables, et un deuxième principe actif qui est choisi dans le groupe constitué par l'acide (plus/moins)-[2-[4-(p-chloro-alpha-phénylbenzyl)-1-pipérazinyl]éthoxy]acétique [DCI : CETIRIZINE] et ses dérivés pharmaceutiquement acceptables.

2. Composition pharmaceutique selon la revendication 1, **caractérisée en ce que** le premier et/ou le deuxième principe actif est sous forme d'un sel, d'un solvate, d'un N-oxyde ou d'un solvate d'un sel ou d'un N-oxyde pharmaceutiquement acceptable.

3. Composition pharmaceutique selon la revendication 1, **caractérisée en ce que** le premier principe actif est choisi dans le groupe constitué par le 3-cyclopropylméthoxy-4-difluorométhoxy-N-(3,5-dichloropyrid-4-yl)benzamide [DCI : ROFLUMILAST], un sel, solvate ou N-oxyde pharmaceutiquement acceptable de celui-ci ou un solvate d'un sel ou d'un N-oxyde pharmaceutiquement acceptable de celui-ci.

4. Composition pharmaceutique selon la revendication 1, **caractérisée en ce que** le premier principe actif est choisi dans le groupe constitué par le 3-cyclopropylméthoxy-4-difluorométhoxy-N-(3,5-dichloropyrid-4-yl)benzamide [DCI : ROFLUMILAST], et un sel, solvate ou N-oxyde pharmaceutiquement acceptable de celui-ci ou un solvate d'un sel ou d'un N-oxyde pharmaceutiquement acceptable de celui-ci et **en ce que** le deuxième principe actif est choisi dans le groupe constitué par l'acide (plus/moins)-[2-[4-(p-chloro-alpha-phénylbenzyl)-1-pipérazinyl]éthoxy]acétique [DCl : CETIRIZINE] et ses dérivés pharmaceutiquement acceptables.

5. Composition pharmaceutique selon l'une quelconque des revendications 1 à 4, qui est une combinaison fixe orale.

6. Utilisation d'une composition pharmaceutique selon l'une quelconque des revendications 1 à 5, dans la fabrication d'un médicament destiné au traitement de maladies respiratoires.

7. Procédé de préparation d'une composition pharmaceutique telle que définie dans l'une quelconque des revendications 1 à 5, qui comprend le mélange du premier principe actif avec le deuxième principe actif.

8. Produit pharmaceutique comprenant, en combinaison, une préparation d'un premier principe actif qui est choisi dans le groupe constitué par le 3-cyclopropylméthoxy-4-difluorométhoxy- N-(3,5- -dichloropyrid-4-yl)benzamide [DCl : ROFLUMILAST] la (-)-cis-9-éthoxy-8-méthoxy-2-méthyl-1,2,3,4,4a,10b-hexahydro-6-(4-diisopropylaminocarbonylphényl)benzo-[c][1,6]naphtyridine [DCl : PUMAFENTRINE] et leurs dérivés pharmaceutiquement acceptables, et une préparation d'un deuxième principe actif qui est choisi dans le groupe constitué par l'acide (plus/moins)-[2-[4-(p-chloro-alpha-phénylbenzyl)-1-pipérazinyl]éthoxy]acétique [DCI : CETIRIZINE] et ses dérivés pharmaceutiquement acceptables, destiné à une utilisation simultanée, séquentielle ou séparée en thérapie.

9. Produit pharmaceutique selon la revendication 8, **caractérisé en ce que** le premier et/ou le deuxième principe actif est sous forme d'un sel, d'un solvate, d'un N-oxyde ou d'un solvate d'un sel ou d'un N-oxyde pharmaceutiquement acceptable.

10. Produit pharmaceutique selon la revendication 8, **caractérisé en ce que** le premier principe actif est choisi dans le groupe constitué par le 3-cyclopropylméthoxy- 4 - difluorométhoxy- N-(3,5-dichloropyrid-4-yl)benzamide [DCl : ROFLUMILAST], un sel, solvate ou N-oxyde pharmaceutiquement acceptable de celui-ci ou un solvate d'un sel ou d'un N-oxyde pharmaceutiquement acceptable de celui-ci.

11. Produit pharmaceutique selon la revendication 8, **caractérisé en ce que** le premier principe actif est choisi dans le groupe constitué par le 3-cyclopropylméthoxy- 4 - difluorométhoxy- N-(3,5-dichloropyrid-4-yl)benzamide [DCl: ROFLUMILAST], et un sel, solvate ou N-oxyde pharmaceutiquement acceptable de celui-ci ou un solvate d'un sel ou d'un N-oxyde pharmaceutiquement acceptable de celui-ci et **en ce que** le deuxième principe actif est choisi dans le groupe constitué par l'acide (plus/moins)-[2-[4-(p-chloro-alpha-phénylbenzyl)-1-pipérazinyl]éthoxy]acétique [DCI : CETIRIZINE] et ses dérivés pharmaceutiquement acceptables.

12. Utilisation d'un produit pharmaceutique selon l'une quelconque des revendications 8 à 11, dans la fabrication d'un médicament destiné au traitement de maladies respiratoires.

13. Kit comprenant une préparation d'un premier principe actif qui est choisi dans le groupe constitué par le 3-cyclopropylméthoxy-4-difluorométhoxy-N-(3,5-dichloropyrid-4-yl)benzamide [DCI: ROFLUMILAST], la (-)-cis-9-éthoxy-8-méthoxy-2-méthyl-1,2,3,4,4a,10b-hexahydro-6-(4-diisopropylaminocarbonylphényl)benzo-[c][1,6] naphtyridine [DCI : PUMAFENTRINE] et leurs dérivés pharmaceutiquement acceptables, une préparation d'un deuxième principe actif qui est choisi dans le groupe constitué par l'acide (plus/moins)-[2-[4-(p-chloro-alpha-phénylbenzyl)-1-pipérazinyl]éthoxy]-acétique [DCI : CETIRIZINE] et ses dérivés pharmaceutiquement acceptables, et des instructions pour l'administration simultanée, séquentielle ou séparée des préparations à un patient en ayant besoin.

14. Kit selon la revendication 13, **caractérisé en ce que** le premier et/ou le deuxième principe actif est sous forme d'un sel, d'un solvate, d'un N-oxyde ou d'un solvate d'un sel ou d'un N-oxyde pharmaceutiquement acceptable.

15. Kit selon la revendication 13, **caractérisé en ce que** le premier principe actif est choisi dans le groupe constitué par le 3-cyclopropylméthoxy-4-difluorométhoxy-N-(3,5-dichloropyrid-4-yl)benzamide [DCl : ROFLUMILAST], un sel, solvate ou N-oxyde pharmaceutiquement acceptable de celui-ci ou un solvate d'un sel ou d'un N-oxyde pharmaceutiquement acceptable de celui-ci.

16. Kit selon la revendication 13, **caractérisé en ce que** le premier principe actif est choisi dans le groupe constitué par le 3-cyclopropylméthoxy-4-difluorométhoxy-N-(3,5-dichloropyrid-4-yl)benzamide [DCl : ROFLUMILAST], et un sel, solvate ou N-oxyde pharmaceutiquement acceptable de celui-ci ou un solvate d'un sel ou d'un N-oxyde pharmaceutiquement acceptable de celui-ci et **en ce que** le deuxième principe actif est choisi dans le groupe constitué par l'acide (plus/moins)-[2-[4-(p-chloro-alpha-phénylbenzyl)-1-pipérazinyl]éthoxy]-acétique [DCI : CETIRIZINE] et ses dérivés pharmaceutiquement acceptables.

17. Utilisation selon l'une quelconque des revendications 6 ou 12, **caractérisée en ce que** la maladie respiratoire est choisie parmi la bronchite, la bronchite obstructive, la bronchite spastique, la bronchite allergique, l'asthme allergique, l'asthme bronchique, la BPCO, la rhinite allergique, la rhinite saisonnière et la rhinite perannuelle.
